(19) Europäisches Patentamt
European Patent Office
Office européen des brevets

(11) **EP 3 352 718 B1**

(12) **EUROPEAN PATENT SPECIFICATION**

(45) Date of publication and mention
of the grant of the patent:
**03.03.2021 Bulletin 2021/09**

(51) Int Cl.:
*A61F 13/49* *(2006.01)*     *A61F 13/511* *(2006.01)*
*A61F 13/532* *(2006.01)*     *A61F 13/551* *(2006.01)*

(21) Application number: **16763173.8**

(22) Date of filing: **31.08.2016**

(86) International application number:
**PCT/US2016/049617**

(87) International publication number:
**WO 2017/053036 (30.03.2017 Gazette 2017/13)**

(54) **ABSORBENT ARTICLES HAVING CURVED CHANNELS**

SAUGFÄHIGE ARTIKEL MIT GEKRÜMMTEN KANÄLEN

ARTICLES ABSORBANTS DOTÉS DE CANAUX INCURVÉS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.09.2015 US 201562221875 P**

(43) Date of publication of application:
**01.08.2018 Bulletin 2018/31**

(73) Proprietor: **The Procter & Gamble Company
Cincinnati, OH 45202 (US)**

(72) Inventors:
• **WILLHAUS, Keith, Richard
  Cincinnati, Ohio 45202 (US)**
• **BIANCHI, Ernesto, Gabriel
  65824 Schwalbach am Taunus (DE)**

(74) Representative: **P&G Patent Germany
Procter & Gamble Service GmbH
Sulzbacher Straße 40
65824 Schwalbach am Taunus (DE)**

(56) References cited:
**WO-A1-00/59430          WO-A2-2015/095514
US-A1- 2014 121 625**

## Description

FIELD OF THE INVENTION

The invention is directed at absorbent articles for personal hygiene that are worn in the crotch region of the wearer such as baby diapers. The invention is for example applicable to taped diapers, training pants and adult incontinence products.

BACKGROUND OF THE INVENTION

Absorbent articles for personal hygiene of the type indicated above are designed to absorb and contain body exudates, in particular large quantity of urine. These absorbent articles comprise several layers, typically a topsheet, a backsheet and in-between an absorbent core, among other layers. The function of the absorbent core is to absorb and retain the exudates for a prolonged amount of time, minimize re-wet to keep the wearer dry and avoid soiling of clothes or bed sheets.

The majority of absorbent cores comprise an absorbent material enclosed within a core wrap. A first type of commonly used absorbent material is a blend of comminuted wood pulp (so-called "air-felt") with superabsorbent polymers (SAP) in particulate form, also called absorbent gelling materials (AGM). Another type of cores having SAP as absorbent material without cellulose fibers (so called "airfelt-free" cores) has been more recently proposed. Fluid-distributing channels extending longitudinally have been proposed for both types of cores.

Through-air bonded carded web (hereinafter "TABCW"), also spelt Thru Air Bonded Carded Web, also referred to as Carded Through Air Bonded Web, are nonwoven webs that have been used in some absorbent articles. TABCW have relatively large pores and fast acquisition time, which make them very useful as topsheet or acquisition layer.

There is a need for continuously improving the fluid handling properties (acquisition, distribution and retention) and the wearing comfort of absorbent articles, while keeping the unit costs as low as possible as these articles are disposable.

SUMMARY OF THE INVENTION

The present invention is for an absorbent article, such as a diaper or a training pant, as indicated in the claims. The absorbent articles of the invention have a wearer-facing side, a garment-facing side, a longitudinal axis and a transversal axis. The absorbent article comprises:

- a topsheet on the wearer-facing side;
- a backsheet on the garment-facing side, the backsheet comprising a liquid impermeable film and optionally a nonwoven attached to the film;
- an absorbent core between the topsheet and the backsheet; and
- optionally an acquisition layer between the absorbent core and the topsheet.

The absorbent core comprises a core wrap having a top side and a bottom side enclosing an absorbent material. The absorbent material comprises a mixture of fibers and absorbent gelling material, with the absorbent material comprising at least 55% by weight of the absorbent material gelling material, preferably from 60% to 85% by weight, more preferably from 60% to 75%. The absorbent core comprises two longitudinally-extending channels substantially free of absorbent material disposed symmetrically on each side of the longitudinal axis. The channels are inwardly curved towards the longitudinal axis so that the channels have a closest distance and a farthest distance from each other as measured in the transversal direction. The closest distance is less than 80% of the farthest distance, in particular wherein the closest distance ranges from 10% to 70% of the farthest distance. At least one of the topsheet, or the acquisition layer if present, is an air-through bonded carded nonwoven.

TABCW have fibers that have been orientated during the carding process. Typically, the fibers are orientated in the longitudinal direction of the articles in which the webs are integrated. While not wishing to be bound by theory, it is believed that this longitudinal orientation of the fibers can preferentially drive the fluid in the same longitudinal direction by capillarity. The curvature of the channels can help partially re-direct the fluid in the transversal direction to a wider area of the core and thus utilize more efficiently the absorbent material of the absorbent core. Furthermore, in the areas of the TABCW corresponding vertically to the channel areas, there is less compaction of the large pores of the carded web. This may also improve the durability and functionality of this web in the areas of the channels.

BRIEF DESCRIPTION OF THE DRAWINGS

Fig. 1 is a schematic perspective view of an exemplary absorbent article of the invention in the form a taped diaper presented in an open and flattened-out configuration with the wearer-facing side facing up;
Fig. 2 is a schematic cross-sectional view of the diaper of Fig. 1;
Fig. 3 is a top view of the absorbent core of the diaper of Fig. 1 shown in isolation;
Fig. 4a shows a cross-section of an absorbent core having a core wrap made of a single substrate;
Fig. 4b shows a cross-section of an alternative absorbent having a core wrap made of two substrates;

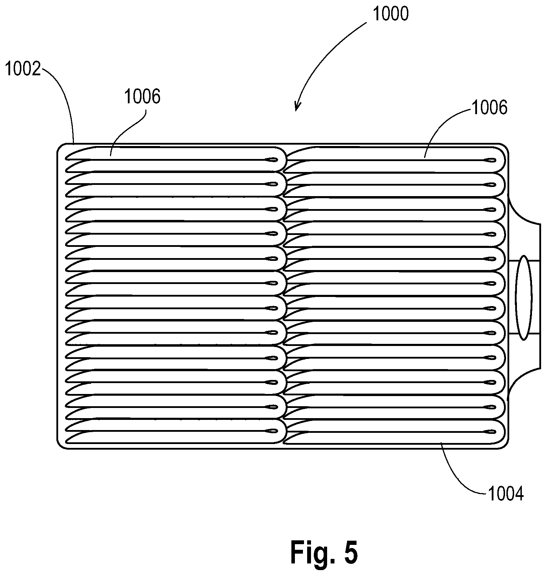

Fig. 5 is a schematic view of a package of absorbent articles of the present invention.

## DETAILED DESCRIPTION OF THE INVENTION

### Introduction

As used herein, the terms "comprise(s)" and "comprising" are open-ended; each specifies the presence of the feature that follows, e.g. a component, but does not preclude the presence of other features, e.g. elements, steps, components known in the art or disclosed herein. These terms based on the verb "comprise" should be read as encompassing the narrower terms "consisting essentially of' which excludes any element, step or ingredient not mentioned which materially affect the way the feature performs its function, and the term "consisting of' which excludes any element, step, or ingredient not specified. Any preferred or exemplary embodiments described below are not limiting the scope of the claims, unless specifically indicated to do so. The words "typically", "normally", "preferably", "advantageously", "in particular" and the likes also qualify features which are not intended to limit the scope of the claims unless specifically indicated to do so.

As used herein, the term "wearer" refers to an incontinent person, which may be an adult, a child, or a baby, and that will wear the absorbent product. The term "user" refers to the caregiver that applies the absorbent article on the wearer. The user may be a parent, a family member in general, a professionally employed caregiver or the wearer him/herself.

The term "nonwoven" is used herein in the usual meaning in the art and means a manufactured sheet, web or batt of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electro spinning, carding and airlaying. The basis weight of nonwoven webs is usually expressed in grams per square meter (g/m$^2$ or gsm).

The invention will now be further illustrated with reference to the embodiments as described in the Figures. For ease of discussion, absorbent articles and their components such as the absorbent core will be discussed with reference to the numerals referred to in these Figures. However it should be understood that these exemplary embodiments and the numerals are not intended to limit the scope of the claims, unless specifically indicated. Nothing in this description should be however considered limiting the scope of the claims unless explicitly indicated otherwise. Dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

### General description of the article 20

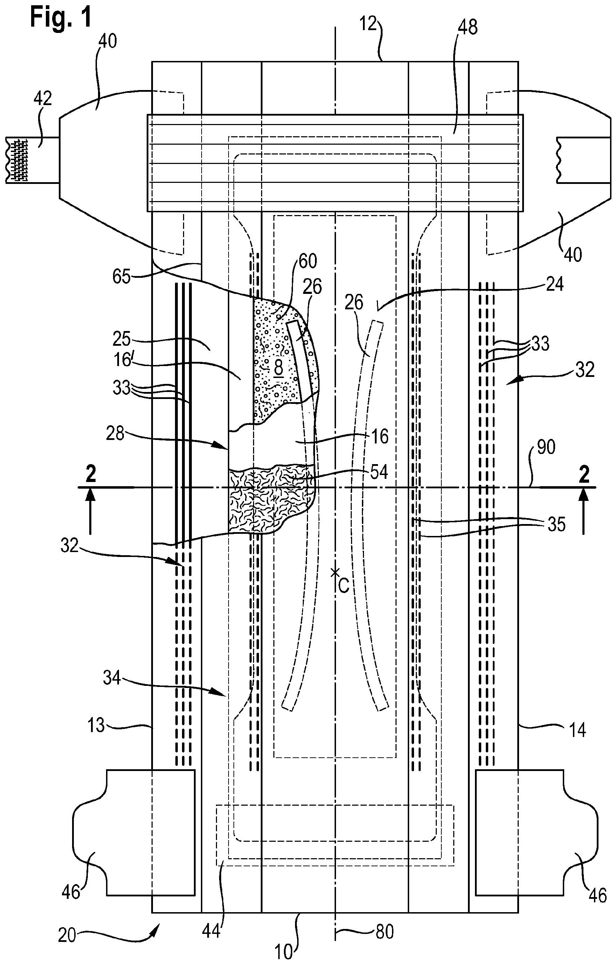

As used herein, the term "absorbent articles" refers to disposable products for personal hygiene such as baby diapers, infant training pants or adult incontinence products and the like which are placed against or in proximity to the body of the wearer to absorb and contain exudates discharged from the body, in particular urine. The absorbent article will now be generally discussed and further illustrated in the form of a baby taped diaper 20 as exemplarily represented in Fig. 1. The diaper is illustrated in a flattened-out configuration with the taped ends 40 opened and the wearer-facing side turned up. An article with side seams instead of re-fastenable tapes such as a training pant may also be represented flattened out by cutting it along its side waists.

The absorbent article has a front edge 10, a back edge 12 and the longitudinally-extending side edges 13, 14. The front edge 10 is placed in use towards the front of the wearer and the back edge 12 forms towards the back of the wearer. When the diaper is closed, the front and back edges form together the waist opening for the wearer. The side edges 13, 14 each forms one of the leg openings. The article has a longitudinal direction and a transverse direction defined by the longitudinal axis 80 and transversal axis 90 respectively. The longitudinal axis 80 extends through the middle of the front and back edges 10, 12 of the article, and thus virtually divides the article in symmetrical left side and right side. The article has a length L along this longitudinal axis between the front and back edges of the article. The transversal axis 90 extends perpendicularly to the longitudinal axis and crosses the longitudinal axis at a position halfway between the front edge and the back edge (L/2 from the front and back edges).

The article comprises on its wearer-facing side a topsheet 24 and a backsheet 25 on its opposite, garment-facing side. Typically the topsheet may be a nonwoven with very good fluid permeability. Typical backsheet comprises a liquid-impermeable film, which may be doubled externally by a softer non-woven layer on its surface. The backsheet film may comprise micropores to make the film vapor-permeable. Examples of topsheet and backsheet will be further discussed below.

The absorbent articles of the invention may advantageously further comprises an acquisition layer 54 (also called distributional layer, acquisition-distribution layer, or secondary-topsheet) placed between the topsheet and the absorbent core. As will be indicated in more detail further below, at least one of the topsheet and/or the acquisition layer is an air-through bonded carded nonwoven. The acquisition layer is typically not as large as the topsheet, and may extend along the whole length of the diaper or be shorter. The acquisition layer may for example be about as wide as the absorbent core underneath (in transversal direction), but it may also be wider or narrower.

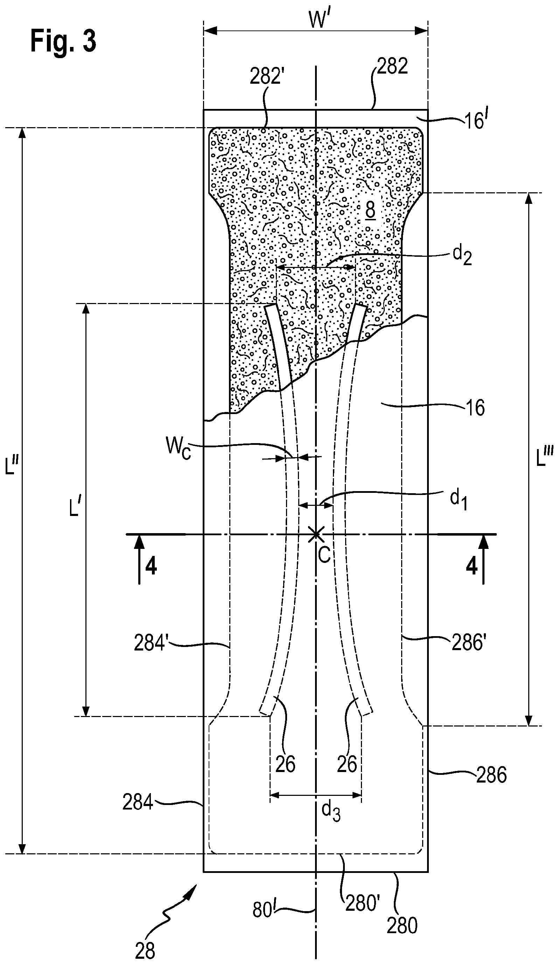

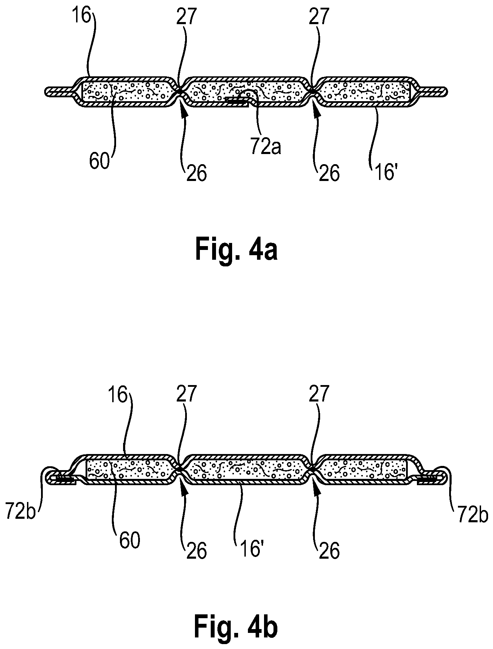

The absorbent core 28 typically comprises a mixture of fibers and superabsorbent polymer particles enclosed in a core wrap. As represented in Fig. 4a, a single substrate material may be completely wrapped around the absorbent material 60 and be bonded to itself by an adhesive core wrap bond 72 along an overlap for example on the bottom side of core wrap. The core wrap may alternatively comprise two separate nonwoven substrates forming the top side and bottom side respectively of the core wrap as shown in Fig. 4b, with one substrate being wider than the other to form flaps which are wrapped around and bonded to the other substrate in a C-wrap configuration. The absorbent core comprises at least two generally longitudinally-extending channels 26 which are substantially free of absorbent material and through which the core wrap is advantageously bonded to itself. The absorbent material 60 defines an absorbent material deposition area 8 within the core wrap as seen from the top of the core. The absorbent material deposition area may be advantageously shaped to define two recesses on each of the longitudinally-extending side edges 284, 286 of the core towards its crotch area similar to a dog-bone or glass-hour shape as illustrated on Fig. 3. The absorbent material deposition area may also be rectangular with straight longitudinal side edges.

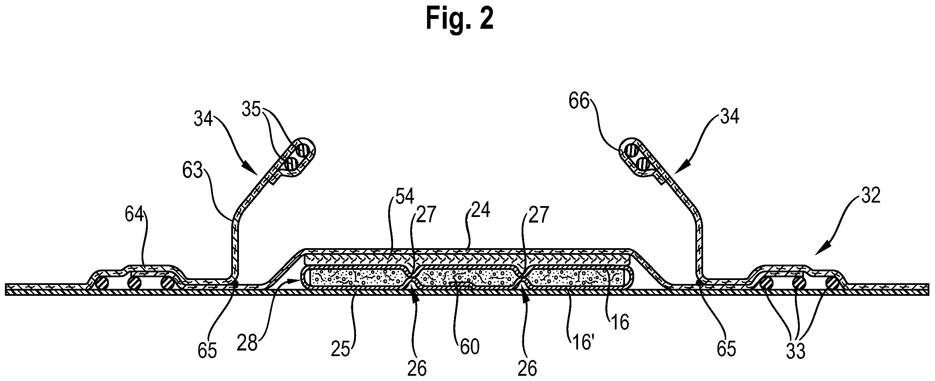

Other layers of the absorbent article are better illustrated in Fig. 2, which shows in cross-section the liquid permeable topsheet 24, the liquid impermeable backsheet 25, the absorbent core 28, the acquisition layer 54 and other typical diaper components. The acquisition layer does not comprise SAP as this may slow the acquisition and distribution of the fluid. The absorbent article may typically comprise a pair of partially upstanding barrier leg cuffs 34 and elasticized gasketing cuffs 32 substantially planar with the chassis. Both types of cuffs are typically joined to the chassis of the absorbent article typically via bonding to the topsheet and/or backsheet. The absorbent article may also comprise a wetness indicator, such as a composition comprising a hot-melt adhesive and pH-indicating agent placed on the internal side of the backsheet film or the external side of the bottom side of the core wrap. The wetness indicator may be a line generally aligned with the longitudinal axis, but it may also comprise several lines or more complex comprising discrete decorative elements such as images of toys, animals etc.. Examples of wetness indicators are further disclosed for example in WO2015/095514 (Laveeta). The wetness indicator may be disposed as to appear from the garment-facing side between the pair of the core's channels. Of course the articles may further comprise any typical components known in the art such as an elastic back elastic waistband, a front elastic waistband, transverse barrier cuff(s), a lotion application, etc....

## Topsheet 24

The topsheet may be any suitable material known in the art for use as a topsheet. The topsheet should be compliant, soft-feeling, and non-irritating to the wearer's skin. Further, at least a portion of the topsheet is liquid permeable, permitting liquids to readily penetrate through its thickness. The topsheet may typically be a nonwoven or an apertured film. An example of topsheet comprises a web of spunbond polypropylene fibers. Typical diaper topsheets have a basis weight of from about 10 to about 28 gsm, in particular between from about 12 to about 18 gsm but other basis weights are possible. Suitable formed film topsheets are for example described in US 3,929,135, US 4,324,246, US 4,342,314, US 4,463,045, and US 5,006,394. Other suitable topsheets may be made in accordance with US 4,609,518 and US 4,629,643.

The topsheet may be three-dimensional, or the article may further comprise an additional three-dimensional material joined to a conventional topsheet and forming part of the wearer-facing layer. Thus additional three-dimensional material may be attached topsheet, typically using an adhesive for example an adhesive having a spiral application pattern, and may be typically shorter in the transversal and in longitudinal direction than the topsheet, but it is not excluded that it may be as long and/or as wide as the topsheet. Such a wearer-facing material may comprise a fibrous support layer and a fibrous projection layer. Examples of such dual layered three-dimensional material and processes to obtain them have been disclosed for example in US2014/0121623A1 (see also US2014/0121621A1, US2014/0121624A1, US2014/0121625A1). The projection layer comprises a plurality of fibrous projections extending from the outer surface of the projection layer in a direction away from the support layer and formed from a first plurality of fibers in the projection layer. The projection layer will be at least partially in contact with the wearer's skin when the article is worn. The projections may be at least partially hollow, but can also be partially filled with fibers from the projection layer and/or the support layer. The wearer-facing material may be a fluid entangled laminate web as indicated in the references above.

Although not shown in the drawings, it is possible and advantageous to bond the topsheet directly or indirectly to the underlying acquisition layer. These layers may be bonded by any known bonding means, such as slot gluing, spiral gluing, fusion point bonding, or otherwise attached.

## Through air-bonded carded web 54

The absorbent article may comprise an acquisition layer 54 between the topsheet and the absorbent core. The acquisition layer may be the only layer between topsheet and core, but it is not excluded that there may be additional layers. Acquisition layer may be also referred to as "distribution layer", "acquisition-distribution layer" or "secondary topsheet". According to the present invention, at least one of the topsheet, or the acquisition layer (if present) is a through-air bonded carded web ("TABCW"). "Bonded carded web" refers to webs that are made from staple fibers that are sent through a combing or carding unit, which breaks apart and aligns the staple fibers in the machine direction to form a generally machine direction-oriented fibrous nonwoven web. This web is then drawn through a heated drum, creating bonds throughout the fabric without applying specific pressure (thru air bonding process). The TABCW material provides a low density, lofty through-air bonded carded web.

The web may in particular have a specific weight basis level at about 15 to about 70 gsm (gram per $m^2$). The TABCW material can for example comprise about 3 to about 10 denier staple fiber. Examples of such TABCW are disclosed in WO2000/71067 (KIM DOO-HONG et al.). TABCW are available directly from all usual suppliers of nonwoven webs for use in absorbent articles, for example Fitesa Ltd or Fiberweb Technical Nonwovens. In a carded nonwoven, the fibers in the web are aligned predominantly in the machine direction and have a more uniform fiber alignment than other nonwovens, which results in greater stability and internal bond strength. The bonding technique chosen influences the fabric's integrity. Through-air bonded carded web have excellent softness, bulk and compressibility, and rapid strike through and good rewet. Synthetic, natural and recycled fibers in a wide range of deniers can be used. Soft PE/PP bicomponent staple fibers may in particular be used.

## Absorbent core 28

As used herein, the term "absorbent core" refers to the component of the article which comprises an absorbent material enclosed in a core wrap and used to absorb and retain most of the liquid exudates. The absorbent core is typically the component of an absorbent article that has the most absorbent capacity of all the components of the absorbent article, and which comprises all, or at least the majority of, superabsorbent polymer (SAP). As used herein, the term "absorbent core" does not include the top sheet, the backsheet and (if present) any acquisition-distribution layer or multilayer system, which is not integral part of the absorbent core. The terms "absorbent core" and "core" are herein used interchangeably.

An exemplary core 28 comprising channels is represented separately in Figs. 3-4 in a dry state outside an absorbent article. Absorbent cores can typically be laid flat on a surface as shown on Fig. 3. Absorbent cores may also be typically thin and conformable, so that they can also be laid on a non-flat surface for example a drum during their making process or stored as a continuous roll of stock material before being converted into an absorbent article. For ease of discussion, the exemplarily absorbent core of Fig. 3 is represented in a flat state and extending in a longitudinal direction 80' and a perpendicular transversal direction. These directions are typically parallel to the corresponding directions of the absorbent article. Unless otherwise indicated, dimensions and areas disclosed herein apply to the core in this flat-out configuration. The same applies to the absorbent article in which the core is integrated.

The absorbent core as delimited by the core wrap 16, 16' is typically rectangular with a front end 280, a back end 282 and two longitudinally extending side edges 284, 286. The core has a width W' as measured in the transversal direction and a length L as measured in the longitudinal direction, from edge to edge including the region of the core wrap which does not enclose the absorbent material. The front end and back end may or may not be sealed. The width and length of the core may vary depending on the intended usage. For baby and infant diapers, the width W' may for example in the range from 40 mm to 200 mm and the length L from 100 mm to 500 mm, as measured along the longitudinal axis 80' of the core. In case the core is not rectangular, the maximum dimension measured along the transversal and longitudinal direction can be used to report the length and width of the core.

The core wrap comprises a top side 16 oriented towards the wearer-facing side of the article and a bottom side 16' oriented towards the garment-facing side of the article. The core wrap may be formed of a single web wrapped around the absorbent material with one longitudinal seal 72a to attach overlapping portions of the substrate to each other, as exemplary shown on Fig. 4a. The top and bottom sides may also be formed by two separate substrates which may be the same or different material (the top layer being for example hydrophillically treated). These two substrates may be partially attached together in particular by gluing the flaps of the wider material to the other material to form two so-called C-wrap seals 72b extending longitudinally of the core as exemplary shown on Fig. 4b. This gluing may be for example provided by two slots of glue. Independent of the construction, the core wrap material may be any suitable material used in the field, typically a nonwoven web, such as a laminate comprising spunbond ("S") or meltblown ("M") layer. For example spunmelt polypropylene nonwovens are suitable, in particular those having a laminate web SMS, or SMMS, or SSMMS, structure, and having a basis weight range of about 5 gsm to 15 gsm. Suitable materials are for example disclosed in US 7,744,576, US 2011/0268932 A1, US 2011/0319848 A1 and US 2011/0250413 A1. It is also not excluded that the core wrap may be partially or entirely formed by layers having an additional function such as the backsheet, the topsheet or an acquisition layer.

The absorbent material in the core may typically comprise fibers mixed with superabsorbent polymer particles. The fibers may typically comprise wood pulp (cellulose) fibers optionally mixed with synthetic fibers. The absorbent material typically comprises from 50% to 90% of superabsorbent polymers (herein abbreviated as "SAP" also referred to as absorbent gelling material) by weight of the absorbent material. The absorbent material may for example comprise at least 55% superabsorbent polymers by weight of the absorbent material, in particular from 60% to 90% superabsorbent polymers by weight of the absorbent material, in particular from 65% to 85% superabsorbent polymers by weight of the absorbent material. It is not excluded that higher amount of SAP may be present, and in some cases it may be possible that the absorbent material comprise little or no cellulose fibers (so called airfelt-free cores).

The term "superabsorbent polymer" refers herein to absorbent material, which may be cross-linked polymer, and that can typically absorb at least 10 times their weight of an aqueous 0.9% saline solution as measured using the Centrifuge Retention Capacity (CRC) test (EDANA method WSP 241.2-05E). The SAP may in particular have a CRC value of more than 20 g/g, or more than 24 g/g, or of from 20 to 50 g/g, or from 20 to 40 g/g, or from 24 to 30 g/g. The SAP may be typically in particulate forms (superabsorbent polymer particles), but it not excluded that other forms of SAP may be used such as a superabsorbent polymer foam for example. It is not excluded that higher amount of SAP may be present, and in some cases it may be possible that the absorbent material comprise little or no cellulose fibers (so called airfelt-free cores).

The absorbent material may comprise as fibers a coform material which is a blend of meltblown fibers and absorbent fibers such as cellulosic fibers that can be formed by air forming a meltblown polymer material while simultaneously blowing air-suspended fibers into the stream of meltblown fibers ("coform"). The coform material can also include other materials, such as superabsorbent materials. Coform materials are further described in US5,508,102 and US5,350,624 (Georger et al.) and 4,100,324 (Anderson).

The absorbent material 60 defines an absorbent material deposition area 8, as seen as in Fig. 4 from above within the plane of the core. The channels 26 are encompassed within this deposition area. The absorbent material deposition area 8 may be advantageously shaped, i.e. it is not rectangular, for example it may have a sand-hour or dog-bone shape as shown in Fig. 3, but other shapes can also be used such as a "T" or "Y". The deposition area 8 typically has a front side 280', a back side 282' and two longitudinally-extending sides 284', 286', each respectively adjacent to the corresponding sides of the core wrap. The length of the deposition area L" is the distance between the front side and the back side of the deposition area. The deposition area preferably shows a tapering along its width in an intermediate position between the front edge and the back edge of the core as illustrated in Fig. 3. In this way, the absorbent material deposition area may have a relatively narrow width in an area of the core intended to be placed in the crotch region of the absorbent article. Having the width of the absorbent material deposition area maximum at the front edge and/or at the back edge of the absorbent material area, and minimum at a longitudinal position between the front edge and the back edge of the absorbent material area may provide better wearing comfort for the wearer. The area of minimum width intermediate the front side 280' and the back side 282' of the absorbent material deposition area, including any transition areas, may for example have a length (L'") which ranges from 10% to 80% of the length of the deposition area as measured in the longitudinal direction.

## Channels 26

The absorbent cores of the invention comprise at least one, and advantageously at least one pair, of longitudinally-extending channels 26. The channels are defined by areas within the absorbent material deposition area 8 that are substantially free of absorbent material. The absorbent core may in particular comprise a pair of channels symmetrically placed relative to the longitudinal axis, but it is not excluded that only one channel may be present, or more than a pair of channels. By "substantially free" it is meant that in the channel areas the basis weight of the absorbent material is at least less than 25%, in particular at least less than 20% or less than 10%, of the average basis weight of the absorbent material in the rest of the absorbent material deposition area. In particular there can be no absorbent material in the channels. Minimal amount such as involuntary contaminations with absorbent material that may occur during the making process are not considered as absorbent material. The channels 26 are advantageously surrounded by the absorbent material, when seen in the plane of the core as seen on Fig. 4, which means that the channels do not extend to any of the edge of the deposition area 8 of the absorbent material. Typically, the smallest distance between a channel 26 and the closest edge of the absorbent material deposition area 8 is at least 5 mm.

The top side 16 of the core wrap may be attached to the bottom side 16' of the core wrap through the channels by one or a plurality of channel bonds 27. Various bonding means can be used such as ultrasonic, heat (fusion), mechanical or adhesive bonding. Ultrasonic bonding may be particular useful in terms of reduced raw material usage and strength of the bond. When the absorbent material swells upon absorbing a liquid, the channel bonds within the channels may remain at least initially attached in the channels. The absorbent material swells in the rest of the core, so that the core wrap forms more marked three-dimensional channels along each channel 26 where the channel bond 27 is present. These channels can distribute an insulting fluid along their length to a wider area of the core and thus provide a quicker fluid acquisition speed and a better utilization of the absorbent capacity of the core. The three-dimensional channels 26 can also provide a deformation of an overlying layer such as a fibrous acquisition layer 54 and provide corresponding ditches in the overlying layer (see WO2014/200794A1). The channel bond 27 may be a continuous bond extending along each of the channels 26, but the channel bond in each channel is typically discontinuous (intermittent) such as series of point bonds.

The following are examples of shape and size of channels, but are not limiting the scope of the invention. In general, the channel bond 27 may have the same outline but be slightly smaller than the channels 26 due to the tolerance required in some manufacturing process. The channels 26 may be present within the crotch region of the article, as defined as being the longitudinally middle third of the article. The absorbent core may also comprise more than two channels, for example at least 3, or at least 4 or at least 5 or at least 6.

The channels extend generally longitudinally, which means that each channel area extends at least as much in the longitudinal direction as in the transverse direction, and typically at least twice as much in the longitudinal direction than in the transverse direction (as measured after projection on the respective axis). The absorbent core, as illustrated in Fig. 3, typically also have a longitudinal axis 80' which is contiguous with the longitudinal axis of the article. The channels 26 may have a length L' projected on the longitudinal axis 80' of the core that is at least 10% of the length L of the absorbent article, in particular from 20% to 80%. The channels 26 may be for example have a length L' of at least 2 cm as measured on the longitudinal axis, or at least 4 cm, 6 cm, 8 cm, or 10 cm, and for example up to 40 cm, or 30 cm. Shorter channels may also be present in the core , for example in the back region or the front region of the core, as seen for example in the Figures of WO2012/170778.

The channels 26 are further inwardly curved (concave) towards the longitudinal axis 80/80', as exemplarily represented in Fig. 3. The channels are separated from each other at their closest by a distance d1, and at their farthest by a distance d2, d3. The farthest distance separating the channels is typically the distance d2 between either the back extremities of the channels, or the distance d3 between the two front extremities of the channels, whichever is the longest. In the example represented in Fig. 3, the channels extend symmetrically towards front and back so that the distance d2 is about equal to d3. The channels are sufficiently curved so that the closest distance d1 is at least less than 85% of the farthest distance d2, d3, preferably wherein the closest distance ranges from 10% to 80% of the farthest distance. This can be summarized by the equation:

$$d1 \leq 0.80 \max (d2, d3)$$

in particular:

$$0.10 \max (d2, d3) \leq d1 \leq 0.70 \max (d2, d3)$$

wherein max (d2, d3) means the largest of d2 or d3 (d2 and d3 can also be about equal).

The radius of curvature may be constant for a channel, or it may vary along its length. The channels may be comprised of a series of generally straight segments approximating a curve (for example three linked segments, the middle one being straight and longitudinally oriented and the two remaining one tilted relative to the longitudinal axis) instead of being a smooth curve as represented. The channels 26 typically do not coincide with the longitudinal axis 80/80' of the article/core. The smallest spacing distance d1 between the channels forming a pair may be for example at least 5 mm, or at least 10 mm, or at least 16 mm. Each channel may have a width Wc along at least part of its length which is at least 2 mm, or at least 3 mm or at least 4 mm, up to for example 20 mm, or 16 mm or 12 mm. The width Wc of each channel 26 may be constant through substantially its whole length or may vary along its length.

Furthermore, in order to reduce the risk of fluid leakages, the channels 26 may advantageously not extend up to any of the edges of the absorbent material deposition area 8, and are therefore surrounded by and fully encompassed within the absorbent material deposition area 8 of the core. Typically, the smallest distance between a channel 26 and the closest edge of the absorbent material deposition area 8 is at least 5 mm.

As the absorbent core absorbs liquid, the depressions formed by these channels will become deeper and more apparent to the eye and the touch from the exterior of the article as the backsheet and topsheet are pushed outwardly by the expending absorbent material. If the channel bond 27 is sufficiently strong and the level of SAP is not too high, it is possible that the channel bonds remain permanent until complete saturation of the absorbent material. On the other hand, the channel bonds may in some cases also restrict the swelling of the absorbent material when the core is substantially loaded. The channel bond 27 may also be designed to open in a controlled manner when exposed to a large amount of fluid. The bonds may thus remain substantially intact at least during a first phase as the absorbent material absorbs a moderate quantity of fluid. In a second phase the channel bonds 27 in the channels can start opening to provide more space for the absorbent material to swell while keeping most of the benefits of the channels such as increased flexibility of the core in transversal direction and fluid management. In a third phase, corresponding to a very high saturation of the absorbent core, a more substantial part of the channel bonds can open to provide even more space for the swelling absorbent material to expand. The strength of the channel bonds 27 within the channels can be controlled for example by varying the number and intensity of the point bonds attaching the two sides of the core wrap and/or the distribution of the superabsorbent material, as more absorbent material will usually causes more swelling and will put more pressure on the bond. The extensibility of the material of the core wrap may also play a role.

**Backsheet 25**

The backsheet may be any backsheet known in the art for absorbent articles. The backsheet may be positioned directly adjacent the garment-facing surface of the absorbent core. The backsheet prevents, or at least inhibits, the exudates absorbed and contained therein from soiling articles such as bedsheets and undergarments. The backsheet is typically impermeable, or at least substantially impermeable, to liquids (e.g., urine). The backsheet may, for example, be or comprise a thin plastic film such as a thermoplastic film having a thickness of about 0.012 mm to about 0.051 mm. Example backsheet films include those manufactured by Tredegar Corporation, based in Richmond, VA, and sold under the trade name CPC2 film. A covering low basis weight nonwoven may be attached to the external surface of the film to provide for a softer touch.

Suitable backsheet materials include breathable materials which permit vapors to escape from the absorbent article while still preventing, or at least inhibiting, exudates from passing through the backsheet. Example breathable materials may include materials such as woven webs, nonwoven webs, composite materials such as film-coated nonwoven webs, microporous films such as manufactured by Mitsui Toatsu Co., of Japan under the designation ESPOIR NO and by Tredegar Corporation of Richmond, VA, and sold under the designation EXAIRE, and monolithic films such as manufactured by Clopay Corporation, Cincinnati, OH under the name HYTREL blend P18-3097.

The film may include at least about 20 weight percent filler particles, for example filler particles that include calcium carbonate, so that wherein the film has been stretched in the machine direction, e.g. to at least about 150 percent, fractures are formed where said filler particles are located. The films may be biaxially stretched at least about 150 percent in the machine direction and a transverse direction to cause fractures to form where said filler particles are located. Breathable films may generally have Water Vapor Transmission Rates (WVTR) in excess of 300 grams per square meter per 24 hours. The WVTR may be measured by the Desiccant Method as indicated in ASTM E96/E96M - 14.

US6,075,179 for example discloses a suitable multilayer film comprising: a core layer made from an extrudable thermoplastic polymer, the core layer having a first exterior surface and a second exterior surface, a first skin layer attached to the first exterior surface of said core layer to form the multilayer film, the multilayer film defining an overall thickness. The first skin layer defines a first skin thickness, and comprising less than about ten percent of said overall thickness. The overall thickness is not exceeding about 30 micrometers and the multilayer film is a liquid barrier and has a WVTR of at least 300 $g/m^2/24$ hours.

The backsheet may further typically comprise a nonwoven on its most external side to improve softness. Exemplary laminates comprising a breathable film and a nonwoven layer are for example disclosed in WO2014/022,362A1, WO2014/022,652A1 and US5,837,352. The nonwoven web may in particular comprise a spunbond nonwoven web and/or a laminate of a spunbond nonwoven web and a meltblown nonwoven web. The laminate may also have a water vapor transmission rate of at least 300 $g/m^2/24$ hours. US5,843,056 for example discloses substantially liquid impermeable, vapor permeable composite backsheet.

**Cuffs 32, 34**

The absorbent articles may typically further comprise components that improve the fit of the article around the legs of the wearer, in particular a pair of barrier leg cuffs 34 and gasketing cuffs 32. The barrier leg cuffs 34 may each be formed by a piece of material, typically a nonwoven, that can be partially raised away and thus stand up from the plane defined by the topsheet, as shown for example in Fig. 2. The material of the barrier leg cuffs may thus comprise a first portion 64 flush with the topsheet and limited inwardly by a proximal edge 65. This first portion 64 may be attached to the topsheet and/or backsheet with an intermittent or continuous fusion bond and/or a glue bond. The barrier leg cuffs 34 further comprise a free-standing portion 63 limited by a distal edge 66, which in use fits at the junction of the thighs with the torso of the wearer, at least in the crotch region of the article. The barrier leg cuffs can provide improved containment of liquids and other body exudates approximately at the junction of the torso and legs of the wearer. Typically, the barrier leg cuffs are formed from a separate material joined to the rest of the article, in particular to the topsheet, but it is not excluded that the barrier leg cuffs can be integral with (i.e. formed from) the topsheet or the backsheet, or any other layer, for example the bottom layer of the core wrap. Typically the material of the barrier leg cuffs may extend through the whole length of the article but is further bonded to the topsheet towards the front edge and back edge of the article so that in these sections the barrier leg cuff material remains flush with the topsheet (tack bonds not shown in Figure 1 for readability). Each barrier leg cuff 34 typically comprises one, two or more elastic strings 35 close to the free standing terminal edge 66.

In addition to the barrier leg cuffs 34, the articles may typically comprise gasketing cuffs 32, which may be present as part of the chassis of the absorbent article. The gasketing cuffs may be at least partially enclosed between the topsheet and the backsheet, or the barrier leg cuffs and the backsheet. The gasketing cuffs may be placed transversally outward relative to the proximal edge 65 of the barrier leg cuffs 34. The gasketing cuffs 32 can provide a better seal around the thighs of the wearer. Usually each gasketing cuff 32 will comprise one or more elastic string or elastic element(s) 33 embedded within the chassis of the diaper, for example between the topsheet and backsheet in the area of the leg openings. These elastic elements 33 may, independently or in combination with the elastics 35 of the barrier leg cuffs, help shaping the absorbent article into a basin shape when put in place on and being worn by the wearer.

Various cuff constructions have been disclosed for in the art and may be used in the present invention. US3,860,003 describes a disposable diaper which provides a contractible leg opening having a side flap and one or more elastic members to provide gasketing cuffs. US4,808,178 and US4,909,803 (Aziz) describe disposable diapers having "stand-up" elasticized flaps (barrier leg cuffs) which improve the containment of the leg regions. US4,695,278 (Lawson) and US4,795,454 (Dragoo) describe disposable diapers having dual cuffs, including gasketing cuffs and barrier leg cuffs. More recently, WO2005/105010 (Ashton) discloses a dual cuff system made of a continuous cuff material. All or a portion of the barrier leg and/or gasketing cuffs may be treated with a lotion.

**Waistband 48**

As illustrated in Fig. 1, the article 20 may also have an elastic back waistband 48 extending transversally adjacent the back waist edge 12 of the article. Such waistbands (also called elastic waist features) may typically comprise a nonwoven substrate and a plurality of elastic strands transversally orientated. Typical waistbands comprise extruded strand elastomer between two layers of spunbond nonwoven; e.g. using PP fibers or bicomponent core/sheath PE/PP or PE/PET fibers. Other types of substrates may be used if desired. Spandex (= Elastane or Lycra®) strands may also be used as elastics between the nonwovens. Other executions of applied waistband consist of elastics stretched in the process and applied transversely to the length of the articles directly sandwiched in between some wearer-facing and some garment-facing material.

The waistbands in cooperation with the other features of the invention may result in absorbent articles having increased comfort, fit, and improved leakage performance for the wearer. The absorbent core with longitudinally-extending channels may provide an increased rigidity in the longitudinal direction when the absorbent material has swollen and presses against the walls of the core wrap defining the channels. This may create further gaps towards the back edge of the article. The stretchable waistband and the stretchable back ears may help solving the problem by providing a better fit. The article may comprise, in addition to the back waistband 48, a front elasticized waistband (not represented). In the following, the description referring to the back waistband may also refer independently to the front waistband, unless specifically indicated otherwise. "Stretchable", "elastic", "elastically extensible", and "elasticized" refer herein to the property of a material and/or an element of a diaper or other disposable absorbent article whereby the material and/or the element can be elongated to at least 150% of its original un-stretched length without rupture or catastrophic failure upon the application of tensioning force and will substantially return to its original length or near its original length after the tension is released.

The waistband 48 typically comprises a laminate of a nonwoven and several elastic strands 50 that are combined with the chassis under some tension. Elastic strands are the most cost effective way to get stretch that exhibits little relax or set over time. Nonwovens are preferred for the exterior of the waist band material because it is breathable and softer than film alternatives, but films may also be used as waistband material. The waistband laminate may further comprise any number of strands are as desired, for example from 2 elastic strands to 40 elastic strands, for example from 4 elastic strands to 26 elastic strands. It is also known that when strands of elastic are combined under strain with other often non-extensible materials and then allowed to relax, they will create a laminate that has gathers of a certain frequency and a resulting basis weight that is higher than the starting materials laid flat. Non-limiting examples of back and front waistbands can be found in WO2012/177400 and WO2012/177401 (Lawson), and US4,515,595, US4,710,189, US5,221,274 and US6,336,922 (Van-Gompel et al.).

The nonwoven material and the elastic strand(s) may be combined under a first strain (Installed Strand Elongation) and the waistband is attached to the article under a second strain (Applied Waistband Strain). The nonwoven material and the elastic strand(s) may be combined with adhesive, mechanical bonds, or any other forms of attachment known in the art.

The Installed Strand Elongation may be greater than about 50%, greater than about 75%, greater than about 100%, greater than about 150%, greater than about 200%, greater than about 225%, greater than about 250%, greater than about 300%, greater than about 350%, greater than about 375%. The installed elongation is the strain at which the elastic is under relative to the second material that it is combined with (e.g. low basis weight nonwoven). For example, if the elastic is stretched from 100 mm to 250 mm when it is combined with the nonwoven, it would be said to be 150% installed elongation or ((250mm/100mm)-1) x 100%. This laminate can then be allowed to relax and will return to about the original 100 mm, but with 250 mm of nonwoven. There can be more than one installed elongation within one waistband laminate if the elastics are strained to a different degree. For example, strand (1) is stretched from 100 mm to 250 mm when combined with the nonwoven or has 150% installed elongation while strand (2) is stretched from 90 mm to 250 mm when combined with the NW or has an installed elongation of about 178%.

The waistband may be applied to the disposable absorbent article at an Applied Waistband Strain of greater than about 30%, greater than about 50%, greater than about 70% as compared to the relaxed length. The waistband may be applied to the disposable absorbent article at an Applied Waistband Strain of less than about 150%, less than about 125%, less than about 100%, less than about 75% as compared to the relaxed length. The Applied Waistband Strain is the strain that the waistband laminate is under when combined with the absorbent article. For example if 100 mm of laminate is stretched to 170 mm when applied it would be considered to be 70% applied waistband strain or ((170mm- 100mm)/100mm x 100%). If a front waistband is present, it may be applied advantageously to the chassis at the same Applied Waistband Strain as the backsheet, or at a different strain.

The waistband may have a length in the direction parallel to the longitudinal axis of the article of greater than about 12 mm, greater than about 15 mm, greater than about 20 mm. The waistband may have a length in the direction parallel to the longitudinal axis of the article f less than about 50mm, less than about 45 mm, less than about 40 mm. The waistband in a relaxed product may have a length in the direction parallel to the transversal axis of the article of greater than about 50mm, greater than about 75 mm, greater than about 100 mm. The length in the direction parallel to the transversal axis of the article of the waistband in a relaxed product may be less than about 300mm, less than about 250 mm, less than about 200 mm. The waistband is typically disposed on the wearer-facing surface of the article, but it is not excluded that the waistband may be on the garment-facing surface of the article. The waistband may be also sandwiched in between the layers of the absorbent article.

The waistband is generally placed adjacent the corresponding waist edge of the article. The distance between the waistband and the edge of the article may be in particular less than 40 mm, in particular the distance between the (back) waistband and the (back) edge of the article may be from 0 mm to 30 mm. The waistband may be attached to the article with adhesive, mechanical bonds, or any other forms of attachment known in the art.

**Other components**

The absorbent articles of the invention can further comprise any other typical components known for the intended purpose of the article. Fig. 1 and Fig. 2 show other typical taped diaper components not further discussed herein such as a fastening system comprising fastening tabs 42 attached to the back ears 40 towards the back edge 12 of the article and cooperating with a landing zone 44 placed towards the front edge 10 of the article. The back ears may be stretchable or not. Fastening features are typically absent from pant-type articles which have a pre-formed side seam, nevertheless the invention may of course also be used in such pant-types articles. As illustrated in Fig. 1, the article may also have a back elastic waistband 48 extending transversally adjacent the back edge of the article. The absorbent article may also comprise other typical components, which are not represented in the Figures, such as a front elastic waistband, transverse barrier element across the topsheet, a wetness indicator between the core and the backsheet that changes appearance when contacted with urine, a lotion application on the topsheet, etc. These components are well-known in the art and will not be further discussed herein. Reference is made to WO2014/093310 where several examples of these components are disclosed in more details.

**Method of making the article** - **Relations between the layers**

The absorbent articles of the invention may be made by any conventional methods known in the art. In particular the articles may be hand-made or industrially produced at high speed. The absorbent core may be made using a standard air laying drums and process adapted to provide channels and bonding through these channels. US2007/250026A1 discloses such an air-laying drum providing a plurality of holes in an unitary absorbent core. The forming drums comprise a plurality of drums forming the holes through which the core wrap can be bonded to itself. The apparatus of this document can be easily modified by changing the plurality of the nubs to at least a pair of curved and longitudinally extending strips to provide the channels of the invention. A method for making absorbent cores with channels in an airfelt-free core process is for example disclosed in WO2012/170798A1.

More generally, adjacent layers within the article will be joined together using conventional bonding method such as adhesive coating via slot coating, spiral gluing, or spraying on the whole or part of the surface of the layer, or thermo-bonding, or pressure bonding or combinations thereof. Most of the bonding between components is for clarity and readability not represented in the Figure. This bonding is exemplarily represented for the channel bond 27 between the core wrap layers within the channels 26 or the C-wrap bond(s) 72 of the core wrap. Other glues or attachments are not represented for clarity and readability but typical bonding between the layers of the article should be considered to be present unless specifically excluded. Adhesives may be typically used to improve the adhesion of the different layers, for example between the backsheet and the core wrap. The glues used may be any standard hotmelt glue as known in the art. For example, the backsheet and the core wrap may be glued using a core-to-backsheet gluing pattern as disclosed in WO2012/170341A1 (Hippe), or a full coverage pattern using several spiral glue applicators. If for example the backsheet is attached by gluing or otherwise to the areas of the core wrap corresponding to the folding guides (not shown), the folding guides may become more visible to the user from the garment-facing side of the article. Any typical hotmelt adhesives may be used. It is also possible to use a printed adhesive layer, for example between the topsheet and absorbent core or liquid management layer, which may be optionally visible through the topsheet, as exemplary disclosed in WO2014/078247.

The articles may be folded and packaged as is known in the art. In particular the articles may be packaged under compression to reduce the space occupied by the package during transport and storage. The package may be for example a plastic bag or a cardboard box. Diapers may typically bi-folded along the transversal axis and the ears folded inwardly before being packaged.

## Packages

The articles may be folded and packaged as is known in the art. The package may be for example a plastic bag or a cardboard box. Diapers may typically bi-folded along the transversal axis and the ears folded inwardly before being packaged. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages. Fig. 6 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

The three-dimensional material described before if present on the article may be particularly resilient to compression so that the articles may be compressed to a certain extent in the package. It is believed that the plurality of relatively closely spaced, relatively small, and relatively pillowy three-dimensional projections may act as springs to resist compression and recover once a compressive force is removed, especially in the areas in the vicinity of the channels. Compression recovery is important in nonwovens or other component layers of absorbent articles, because such articles are typically packaged and folded in compressed conditions. Manufacturers of personal care products desire to retain most, if not all of the as-made caliper for aesthetic and performance purposes. Furthermore, the channels being substantially material-free contribute to an unexpected, beneficial improvement in compression recovery as they provide spacing for at least some of the three-dimensional projections to nest during storage and transport.

The articles of the inventions may be packaged compressed at an In-Bag Compression Rate of at least 10%, in particular of from 10% to 50%, in particular from 20% to 40%. The "In-Bag Compression Rate" as used herein is one minus the height of a stack of 10 folded articles measured while under compression within a bag ("In-Bag Stack Height") divided by the height of a stack of 10 folded articles of the same type before compression, multiplied by 100; i.e. (1-In-Bag Stack Height/stack height before compression) * 100, reported as a percentage. Of course, the stack in the bag does not need to have exactly 10 articles, rather the value measured for the height of stack of article in the package is divided by the number of articles in the stack and then multiplied by 10. The method used to measure the In-Bag Stack Height is described in further details in the Test Procedures. The articles before compression may be typically sampled from the production line between the folding unit and the stack packing unit. The stack height before compression is measured by taking 10 articles before compression and packing, and measuring their stack height as indicated for the IBSH.

Packages of the absorbent articles of the present disclosure may in particular have an In-Bag Stack Height of less than 110 mm, or less than 105 mm, or less than 100 mm, or less than 95 mm, or less than 90 mm according to the In-Bag Stack Height Test described below. The In-Bag Stack Height may be at least 70 mm, at least 72 mm, at least 74 mm, at least 80mm, according to the In-Back Stack Height Test described herein, specifically reciting all 0.1 mm increments within the specified ranges and all ranges formed therein or thereby, such as from 70 mm to 110 mm, from 75 mm to 110 mm, from 80 mm to 110 mm, from 80 mm to 105 mm, or from 80 mm to 100 mm.

## TEST PROCEDURES

### In-Bag Stack Height Test

The in-bag stack height of a package of absorbent articles is determined as follows:

**Equipment:** A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

**Test Procedure:** Absorbent article packages are equilibrated at 23 ± 2 °C and 50 ± 5% relative humidity prior to measurement. The horizontal sliding plate is raised and an absorbent article package is placed centrally under the horizontal sliding plate in such a way that the absorbent articles within the package are in a horizontal orientation. Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

**Claims**

1. An absorbent article (20), such as a diaper or a training pant, having a wearer-facing side, a garment-facing side, a longitudinal axis (80) and a transversal axis (90); the absorbent article comprising:

   - a topsheet (24) on the wearer-facing side;
   - a backsheet (25) on the garment-facing side, the backsheet comprising a liquid impermeable film and optionally a nonwoven attached to the film;
   - an absorbent core (28) between the topsheet and the backsheet;
   - optionally an acquisition layer (54) between the absorbent core and the topsheet;

   wherein the absorbent core comprises a core wrap having a top side (16) and a bottom side (16') enclosing an absorbent material (60), wherein the absorbent material comprises a mixture of fibers and superabsorbent polymers, with the absorbent material comprising at least 55% of superabsorbent polymers by weight of absorbent material, in particular from 60% to 85% by weight of superabsorbent polymers by weight of absorbent material; wherein the absorbent core comprises two longitudinally-extending channels (26) substantially free of absorbent material disposed symmetrically on each side of the longitudinal axis; **characterized in that** the channels are inwardly curved towards the longitudinal axis so that the channels have a closest distance (d1) and a farthest distance (d2, d3) from each other as measured in the transversal direction, wherein the closest distance is less than 80% of the farthest distance, preferably wherein the closest distance ranges from 10% to 70% of the farthest distance; and
   at least one of the topsheet or the acquisition layer (54), if present, is an air-through bonded carded nonwoven.

2. An absorbent core according to claim 1, wherein the absorbent material defines an absorbent material deposition area (8) within the core wrap; wherein the absorbent material area comprises a front side (280'), a back side (282') and two longitudinal sides (284', 286') and this absorbent material area is shaped so that its width as measured in the transversal direction varies along the position on the longitudinal axis; and the width of the absorbent material area is minimum at a longitudinal position intermediate the front side and the back side of the deposition area.

3. An absorbent core according to claim 2, wherein the width of the deposition area is about the same at the front side and the back side of the deposition area, and the area of minimum width intermediate the front side and back side, including any transition areas, has a length (L''') which ranges from 10% to 80% of the length of the deposition area (L'') as measured in the longitudinal direction.

4. An absorbent article according to any of the preceding claims, wherein the air-through bonded carded nonwoven has a basis weight ranging from 5 gsm to 200 gsm, in particular from 10 gsm to 150 gsm.

5. An absorbent article according to any of the preceding claims, wherein the top side and the bottom side are bonded through the channels, in particular wherein the top side and the bottom side of the core wrap are nonwovens which are non-adhesively bonded through the channels, in particular by ultrasonic, pressure and/or heat-bonding.

6. An absorbent article according to any of the preceding claims, comprising a wetness indicator visible from the garment-facing side of the article, in particular wherein the wetness indicator is a composition comprising a hot-melt adhesive and a pH-indicator disposed between the absorbent core and the backsheet and is visually disposed between the channels when seen from the garment-facing side.

7. An absorbent article according to any of the preceding claims, wherein each of the channel has a length (L') projected on the longitudinal axis (80) of the article which is at least 10% of the length (L") of the absorbent material area, in particular from 30% to 80% of the length of the absorbent material area.

8. An absorbent article according to any of the preceding claims, wherein each of the channels have at least in some of their areas a width (Wc) of at least 2 mm, in particular from 2 mm to 20 mm.

9. An absorbent article according to the preceding claim, wherein the core wrap is formed by a single substrate which is C-wrapped around the absorbent material so that the bottom side or top side of the core wrap is formed by partially overlapping flaps of the substrate, or alternatively wherein the core wrap comprise a first substrate forming the top side or the bottom side of the core wrap and a second substrate at least partially forming the other side of the core wrap, wherein the first substrate is larger than the second substrate so that the first substrate can be folded over the second substrate to form a dual substrates C-wrap.

10. An absorbent article according to any of the preceding claims, wherein the topsheet is a spunbond nonwoven and the acquisition layer is an air-through bonded carded nonwoven.

11. An absorbent article according to any of the preceding claims, wherein the topsheet and the acquisition layer are attached together by a plurality of fusion bonds.

12. An absorbent article according to any of the preceding claims, comprising a three-dimensional material on the wearer-facing side.

13. An absorbent article according to any of the preceding claims, wherein the three-dimensional material is disposed over the topsheet, and the three-dimensional material comprises:

   a. a fibrous support layer having an inner surface and an opposed outer surface;
   b. a fibrous projection layer having an inner surface and an opposed outer surface, the outer surface of the support layer being in contact with the inner surface of the projection layer; and
   c. a plurality of hollow projections formed from a first plurality of fibers in the projection layer, the hollow projections extending from the outer surface of the projection layer in a direction away from the support layer.

14. An absorbent article according to any of the preceding claims, further comprising an elasticized back waistband (48).

15. A package comprising a plurality of the absorbent articles according to any of the preceding claims, wherein the package has an In-Bag Stack Height of from 70 mm to 110 mm.

**Patentansprüche**

1. Absorptionsartikel (20), wie eine Windel oder eine Übungshose, mit einer trägerseitigen Seite, einer zum Kleidungsstück zeigenden Seite, einer Längsachse (80) und einer Querachse (90); wobei der Absorptionsartikel umfasst:

   - eine Oberschicht (24) auf der trägerseitigen Seite;
   - eine Unterschicht (25) auf der zum Kleidungsstück zeigenden Seite, wobei die Unterschicht eine flüssigkeitsundurchlässige Folie und wahlweise ein an der Folie befestigtes Vlies umfasst;
   - einen Absorptionskern (28) zwischen der Oberschicht und der Unterschicht;
   - wahlweise eine Aufnahmeschicht (54) zwischen dem Absorptionskern und der Oberschicht;

   wobei der Absorptionskern eine Kernumwicklung mit einer Oberseite (16) und einer Unterseite (16') umfasst, die ein Absorptionsmaterial (60) umschließen, wobei das Absorptionsmaterial eine Mischung aus Fasern und Superabsorber-Polymeren umfasst, wobei das Absorptionsmaterial nach Gewicht des Absorptionsmaterials mindestens 55 % Superabsorber-Polymere, insbesondere 60 % bis 85 % Superabsorber-Polymere nach Gewicht des Absorptionsmaterials, umfasst;
   wobei der Absorptionskern zwei sich in Längsrichtung erstreckende Kanäle (26) umfasst, die im Wesentlichen frei von Absorptionsmaterial sind und symmetrisch auf jeder Seite der Längsachse angeordnet sind;
   **dadurch gekennzeichnet, dass** die Kanäle nach innen in Richtung der Längsachse gekrümmt sind, sodass die Kanäle einen kürzesten Abstand (d1) und einen weitesten Abstand (d2, d3) voneinander aufweisen, wie in der Querrichtung gemessen, wobei der kürzeste Abstand weniger als 80 % des weitesten Abstands beträgt, wobei der kürzeste Abstand vorzugsweise von 10 % bis 70 % des weitesten Abstands reicht; und
   mindestens eine der Oberschicht oder der Aufnahmeschicht (54), falls vorhanden, ein luftdurchleitungsgebundenes kardiertes Vlies ist.

2. Absorptionskern nach Anspruch 1, wobei das Absorptionsmaterial einen Absorptionsmaterial-Ablagebereich (8) innerhalb der Kernumwicklung definiert; wobei der Absorptionsmaterialbereich eine Vorderseite (280'), eine Rückseite (282') und zwei Längsseiten (284', 286') umfasst und dieser Absorptionsmaterialbereich so geformt ist, dass seine wie in der Querrichtung gemessene Breite entlang der Position auf der Längsachse variiert; und die Breite des Absorptionsmaterialbereichs an einer Längsposition zwischen der Vorderseite und der Rückseite des Ablagebereichs minimal ist.

3. Absorptionskern nach Anspruch 2, wobei die Breite des Ablagebereichs an der Vorderseite und der Rückseite des Ablagebereichs etwa gleich ist und der Bereich minimaler Breite zwischen der Vorderseite und der Rückseite, einschließlich jeglicher Übergangsbereiche, eine Länge (L''') aufweist, die von 10 % bis 80 % der Länge des Ablagebereichs (L'), wie in der Längsrichtung gemessen, reicht.

4. Absorptionsartikel nach einem der vorstehenden Ansprüche, wobei das luftdurchleitungsgebundene kardierte Vlies ein Flächengewicht aufweist, das von 5 g/m$^2$ bis 200 g/m$^2$, insbesondere von 10 g/m$^2$ bis 150 g/m$^2$ reicht.

5. Absorptionsartikel nach einem der vorstehenden Ansprüche, wobei die Oberseite und die Unterseite durch die Kanäle gebunden sind, wobei insbesondere die Oberseite und die Unterseite der Kernumwicklung Vliese sind, die nicht durch Haftwirkung durch die Kanäle gebunden sind, insbesondere durch Ultraschall-, Druck- und/oder Wärmebinden.

6. Absorptionsartikel nach einem der vorstehenden Ansprüche, umfassend einen Feuchtigkeitsindikator, der von der zum Kleidungsstück zeigenden Seite des Artikels aus sichtbar ist, wobei insbesondere der Feuchtigkeitsindikator eine Zusammensetzung ist, die einen Heißkleber und einen pH-Indikator umfasst, der zwischen dem Absorptionskern und der Unterschicht angeordnet ist und, wenn von der zum Kleidungsstück zeigende Seite aus gesehen, visuell zwischen den Kanälen angeordnet ist.

7. Absorptionsartikel nach einem der vorstehenden Ansprüche, wobei jeder des Kanals eine auf die Längsachse (80) des Artikels projizierte Länge (L') aufweist, die mindestens 10 % der Länge (L'') des Absorptionsmaterialbereichs, insbesondere 30 % bis 80 % der Länge des Absorptionsmaterialbereichs beträgt.

8. Absorptionsartikel nach einem der vorstehenden Ansprüche, wobei jeder der Kanäle zumindest in einigen seiner Bereiche eine Breite (Wc) von mindestens 2 mm, insbesondere von 2 mm bis 20 mm aufweist.

9. Absorptionsartikel nach dem vorstehenden Anspruch, wobei die Kernumwicklung durch ein einzelnes Substrat gebildet ist, das um das Absorptionsmaterial herum C-gewickelt ist, sodass die Unterseite oder Oberseite der Kernumwicklung durch teilweise überlappende Klappen des Substrats gebildet ist, oder wobei alternativ die Kernumwicklung ein erstes Substrat, das die Oberseite oder die Unterseite der Kernumwicklung bildet, und ein zweites Substrat, das zumindest teilweise die andere Seite der Kernumwicklung bildet, umfasst, wobei das erste Substrat größer als das zweite Substrat ist, sodass das erste Substrat über das zweite Substrat gefaltet werden kann, um eine Doppelsubstrat-C-Umwicklung zu bilden.

10. Absorptionsartikel nach einem der vorstehenden Ansprüche, wobei die Oberschicht ein Spinnvlies ist und die Aufnahmeschicht ein luftdurchleitungsgebundenes kardiertes Vlies ist.

11. Absorptionsartikel nach einem der vorstehenden Ansprüche, wobei die Oberschicht und die Aufnahmeschicht durch eine Vielzahl von Schmelzbindungen aneinander befestigt sind.

12. Absorptionsartikel nach einem der vorstehenden Ansprüche, umfassend ein dreidimensionales Material auf der trägerseitigen Seite.

13. Absorptionsartikel nach einem der vorstehenden Ansprüche, wobei das dreidimensionale Material über der Oberschicht angeordnet ist und das dreidimensionale Material Folgendes umfasst:

a. eine faserige Trägerschicht mit einer Innenoberfläche und einer gegenüberliegenden Außenoberfläche;
b. eine faserige Vorsprungsschicht mit einer Innenoberfläche und einer gegenüberliegenden Außenoberfläche, wobei die Außenoberfläche der Trägerschicht in Kontakt mit der Innenoberfläche der Vorsprungsschicht steht; und
c. eine Vielzahl von hohlen Vorsprüngen, die aus einer ersten Vielzahl von Fasern in der Vorsprungsschicht gebildet sind, wobei sich die hohlen Vorsprünge von der Außenoberfläche der Vorsprungsschicht in einer Richtung weg von der Trägerschicht erstrecken.

14. Absorptionsartikel nach einem der vorstehenden Ansprüche, ferner umfassend ein elastifiziertes rückseitiges Taillenband (48).

15. Packung, umfassend eine Vielzahl von Absorptionsartikeln nach einem der vorstehenden Ansprüche, wobei die Packung eine Stapelhöhe im Beutel von 70 mm bis 110 mm aufweist.

**Revendications**

1. Article absorbant (20), tel qu'une couche ou une culotte de propreté, ayant un côté tourné vers le porteur, un côté tourné vers le vêtement, un axe longitudinal (80) et un axe transversal (90) ; l'article absorbant comprenant :

   - une feuille de dessus (24) sur le côté tourné vers le porteur ;
   - une feuille de fond (25) sur le côté tourné vers le vêtement, la feuille de fond comprenant un film imperméable aux liquides et facultativement un nontissé joint au film ;
   - une âme absorbante (28) entre la feuille de dessus et la feuille de fond ;
   - facultativement une couche d'acquisition (54) entre l'âme absorbante et la feuille de dessus ;

   dans lequel l'âme absorbante comprend une enveloppe d'âme ayant un côté supérieur (16) et un côté inférieur (16') renfermant un matériau absorbant (60), dans lequel le matériau absorbant comprend un mélange de fibres et de polymères superabsorbants, avec le matériau absorbant comprenant au moins 55 % de polymères superabsorbants en poids de matériau absorbant, en particulier de 60 % à 85 % en poids de polymères superabsorbants en poids de matériau absorbant ;
   dans lequel l'âme absorbante comprend deux canaux s'étendant longitudinalement (26) essentiellement dépourvus de matériau absorbant disposés symétriquement sur chaque côté de l'axe longitudinal ; **caractérisé en ce que** les canaux sont courbés vers l'intérieur vers l'axe longitudinal de sorte que les canaux ont une distance la plus proche (d1) et une distance la plus éloignée (d2, d3) l'un de l'autre telle que mesurée dans la direction transversale, dans lequel la distance la plus proche est inférieure à 80 % de la distance la plus éloignée, de préférence dans lequel la distance la plus proche va de 10 % à 70 % de la distance la plus éloignée ; et
   au moins l'une parmi la feuille de dessus ou la couche d'acquisition (54), si elle est présente, est un nontissé cardé lié par soufflage d'air.

2. Âme absorbante selon la revendication 1, dans laquelle le matériau absorbant définit une zone de dépôt de matériau absorbant (8) dans l'enveloppe d'âme ; dans laquelle la zone de matériau absorbant comprend un côté avant (280'), un côté arrière (282') et deux côtés longitudinaux (284', 286') et cette zone de matériau absorbant est formée de sorte que sa largeur telle que mesurée dans la direction transversale varie le long de la position sur l'axe longitudinal ; et la largeur de la zone de matériau absorbant est au minimum à une position longitudinale entre le côté avant et le côté arrière de la zone de dépôt.

3. Âme absorbante selon la revendication 2, dans laquelle la largeur de la zone de dépôt est environ la même sur le côté avant et le côté arrière de la zone de dépôt, et la zone de largeur minimum entre le côté avant et le côté arrière, incluant l'une quelconque des zones de transition, a une longueur (L''') qui va de 10 % à 80 % de la longueur de la zone de dépôt (L'') telle que mesurée dans la direction longitudinale.

4. Article absorbant selon l'une quelconque des revendications précédentes, dans lequel le nontissé cardé lié par soufflage d'air a une masse surfacique allant de 5 g/m$^2$ à 200 g/m$^2$, en particulier de 10 g/m$^2$ à 150 g/m$^2$.

5. Article absorbant selon l'une quelconque des revendications précédentes, dans lequel le côté supérieur et le côté inférieur sont liés à travers les canaux, en particulier dans lequel le côté supérieur et le côté inférieur de l'enveloppe d'âme sont des nontissés qui sont liés de manière non adhésive à travers les canaux, en particulier par liaison par ultrasons, pression et/ou chaleur.

6. Article absorbant selon l'une quelconque des revendications précédentes, comprenant un indicateur d'humidité visible du côté tourné vers le vêtement de l'article, en particulier dans lequel l'indicateur d'humidité est une composition comprenant un adhésif thermofusible et un indicateur de pH disposé entre l'âme absorbante et la feuille de fond et est disposé visuellement entre les canaux lorsqu'ils sont vus du côté tourné vers le vêtement.

7. Article absorbant selon l'une quelconque des revendications précédentes, dans lequel chacun du canal a une longueur (L') faisant saillie sur l'axe longitudinal (80) de l'article qui est au moins 10 % de la longueur (L'') de la zone de matériau absorbant, en particulier de 30 % à 80 % de la longueur de la zone de matériau absorbant.

8. Article absorbant selon l'une quelconque des revendications précédentes, dans lequel chacun des canaux ont au moins dans certaines de leurs zones une largeur (Wc) d'au moins 2 mm, en particulier de 2 mm à 20 mm.

9. Article absorbant selon la revendication précédente, dans lequel l'enveloppe d'âme est formée par un substrat unique qui est enroulé en C autour du matériau absorbant de sorte que le côté inférieur ou côté supérieur de l'enveloppe d'âme est formé par des rabats du substrat se chevauchant partiellement, ou alternativement dans lequel l'enveloppe d'âme comprend un premier substrat formant le côté supérieur ou le côté inférieur de l'enveloppe d'âme et un second substrat formant au moins partiellement l'autre côté de l'enveloppe d'âme, dans lequel le premier substrat est plus grand que le second substrat de sorte que le premier substrat peut être plié sur le second substrat pour former une enveloppe en C de substrats doubles.

10. Article absorbant selon l'une quelconque des revendications précédentes, dans lequel la feuille de dessus est un nontissé filé-lié et la couche d'acquisition est un nontissé cardé lié par soufflage d'air.

11. Article absorbant selon l'une quelconque des revendications précédentes, dans lequel la feuille de dessus et la couche d'acquisition sont jointes ensemble par une pluralité de liaisons de fusion.

12. Article absorbant selon l'une quelconque des revendications précédentes, comprenant un matériau tridimensionnel sur le côté tourné vers le porteur.

13. Article absorbant selon l'une quelconque des revendications précédentes, dans lequel le matériau tridimensionnel est disposé sur la feuille de dessus, et le matériau tridimensionnel comprend :

a. une couche de support fibreuse ayant une surface interne et une surface externe opposée ;
b. une couche en saillie fibreuse ayant une surface interne et une surface externe opposée, la surface externe de la couche de support étant en contact avec la surface interne de la couche en saillie ; et
c. une pluralité de saillies creuses formées à partir d'une première pluralité de fibres dans la couche en saillie, les saillies creuses s'étendant à partir de la surface externe de la couche en saillie dans une direction à l'écart de la couche de support.

14. Article absorbant selon l'une quelconque des revendications précédentes, comprenant en outre une ceinture arrière élastifiée (48).

15. Emballage comprenant une pluralité des articles absorbants selon l'une quelconque des revendications précédentes, dans lequel l'emballage a une hauteur de pile en sachet de 70 mm à 110 mm.

**Fig. 1**

EP 3 352 718 B1

# Fig. 2

**Fig. 3**

**Fig. 4a**

**Fig. 4b**

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015095514 A, Laveeta
- US 3929135 A
- US 4324246 A
- US 4342314 A
- US 4463045 A
- US 5006394 A
- US 4609518 A
- US 4629643 A
- US 20140121623 A1
- US 20140121621 A1
- US 20140121624 A1
- US 20140121625 A1
- WO 200071067 A, KIM DOO-HONG
- US 7744576 B
- US 20110268932 A1
- US 20110319848 A1
- US 20110250413 A1
- US 5508102 A
- US 5350624 A, Georger
- US 4100324 A, Anderson
- WO 2014200794 A1
- WO 2012170778 A
- US 6075179 A
- WO 2014022362 A1
- WO 2014022652 A1
- US 5837352 A
- US 5843056 A
- US 3860003 A
- US 4808178 A
- US 4909803 A, Aziz
- US 4695278 A, Lawson
- US 4795454 A, Dragoo
- WO 2005105010 A, Ashton
- WO 2012177400 A
- WO 2012177401 A, Lawson
- US 4515595 A
- US 4710189 A
- US 5221274 A
- US 6336922 B, VanGompel
- WO 2014093310 A
- US 2007250026 A1
- WO 2012170798 A1
- WO 2012170341 A1, Hippe
- WO 2014078247 A